(19) Europäisches Patentamt
European Patent Office
Office européen des brevets

(11) EP 0 457 381 B1

(12) EUROPEAN PATENT SPECIFICATION

(45) Date of publication and mention of the grant of the patent: **06.03.1996 Bulletin 1996/10**

(51) Int Cl.$^6$: **C07D 501/60**

(21) Application number: **91200943.8**

(22) Date of filing: **19.04.1991**

# (54) Delta 2-cephem sulphones

Delta-2-Cephemsulfone

Delta-2-céphem sulfones

(84) Designated Contracting States:
**DE GB IT**

(30) Priority: **16.05.1990 GB 9010941**

(43) Date of publication of application:
**21.11.1991 Bulletin 1991/47**

(73) Proprietor: **PHARMACIA S.p.A.**
**I-20152 Milano (IT)**

(72) Inventors:
- **Bissolino, Pier Luigi**
  **I-27020 S. Giorgio Lomellina (Pavia) (IT)**
- **Alpegiani, Marco**
  **I-20132 Milan (IT)**
- **Perrone, Ettore**
  **I-20010 Boffalora Ticino (Milan) (IT)**
- **Cassinelli, Giuseppe**
  **I-27058 Voghera (Pavia) (IT)**

(74) Representative: **Ferrario, Vittorino et al**
**Pharmacia S.p.A.**
**Patent Department**
**Via Bisceglie, 104**
**I-20152 Milano (IT)**

(56) References cited:
**WO-A-90/07111**
**DE-A- 1 937 016**
**US-A- 4 208 516**
**US-A- 4 291 160**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 457 381 B1

## Description

The present invention relates to cephem sulphones, their preparation, and to pharmaceutical and veterinary compositions containing them.

$\Delta^3$-Cephem sulphones, in particular some 1,1-dioxo-$\Delta^3$-cepem-4-carboxylic esters having the following framework:

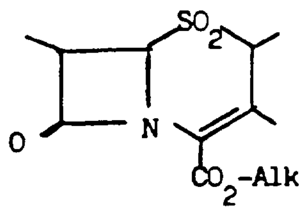

have been found to act as potent inhibitors of human leukocyte elastase (HLE) (J.B. Doherty et al., Nature 1986, 322:192). These were obtained by oxidizing the corresponding $\Delta^3$-cephem.

$\Delta^2$-Cephem sulphones have never been described. Upon oxidation at sulphur, $\Delta^2$-cephem-4-carboxylic esters having the following framework:

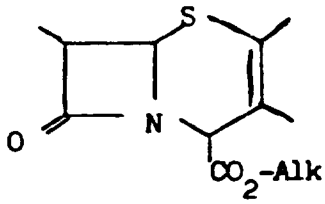

are converted to the $\Delta^3$-derivatives, which are thermodynamically more stable when the sulphur atom is oxidized (C.F. Murphy and J.A. Webber, in "Cephalosporins and Penicillins, Chemistry and Biology", E.H. Flynn ed., Academic Press 1972, page 148-150, and references therein).

According to the invention there is provided a compound of the formula (I), and the pharmaceutically and veterinary acceptable salts thereof:

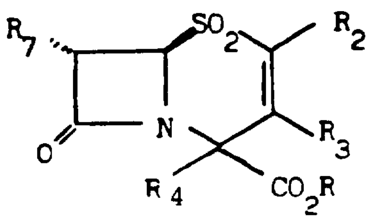

I

wherein $R_7$ is a halogen atom or $C_1$-$C_4$ alkoxy group;
$R_4$ is a substituted alkyl group CHR'-CHR"Y, or an alkenyl group CR'=CHY, wherein R' and R" each represents, independently, hydrogen atom or substituted with free carboxy group $C_1$-$C_7$ straight or branched alkyl, phenyl or benzyl group, or R' and R" taken together with the carbon atoms to which they are attached, form a five or six-membered ring of the formulae

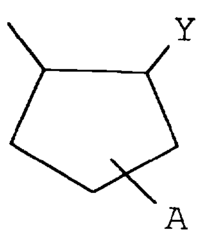

or

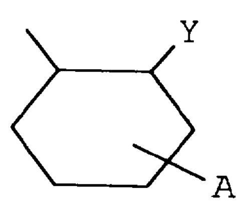

wherein Y is

- acyl C(O)R' wherein R' is as defined above;
- alkoxycarbonyl or benzyloxycarbonyl C(O)OR' wherein R' is as defined above;
- alkylsulphinyl, phenylsulphinyl or benzylsulphinyl S(O)R' wherein R' is as defined above,
- alkylsulphonyl, phenylsulphonyl or benzylsulphonyl $S(O)_2R'$ wherein R' is as defined above;

- carbamoyl C(O)NR'R" wherein R' and R" are as defined above, or wherein R' and R" together with the nitrogen atom to which they are attached, form a pyrrolidine or piperidine ring;
- aminosulphonyl $S(O)_2NR'R"$ wherein R' and R" are as defined above, or wherein R' and R", together with the nitrogen atom, to which they are attached, form a pyrrolidine or piperidine ring;
- nitro; and
- cyano;

and A is hydrogen or a carboxy group; R is hydrogen, a $C_1$-$C_7$ straight or branched alkyl, p-methoxybenzyl, benzyl or diphenylmethyl group; R3 is methyl or a C1-C4 alkoxymethyl group; and R2 is hydrogen or a group R4 as defined above. Δ2-Cephem sulphones provided by the present invention are characterised by high inhibitory activity on HLE. They do not require specific activating groups at $C_3$, such as acetoxymethyl or heterocyclylthiomethyl, as reported by Doherty et al., for Δ3-cephem sulphones (ref. cit.).

They can be used to make medicaments useful to prevent or arrest the progression of diseases caused by proteolytic degradation of tissues, in particular lungs and connective tissues, reduce inflammation and fever, and relieve pain. Such diseases are emphysema, acute respiratory distress syndrome, bronchial inflammation, rheumatoid arthritis, osteoarthritis, infectious arthritis, rheumatic fever, spondylitis, gout, lupus, psoriasis and the like.

In particular, in formula (I), $R_7$ is chloro or methoxy. Preferred examples of $C_1$-$C_7$ straight or branched alkylgroups include methyl, ethyl, i-propyl and tert-butyl groups. Preferably Y is an acetyl, pivaloyl, benzoyl, phenylacetyl, methoxycarbonyl, tert-butoxycarbonyl, benzyloxycarbonyl, carboxy, cyano, nitro, mesyl, tosyl, phenylsulfonyl, tert-butylsulfonyl or benzylsulfonyl group or a carbamoyl derivative of formula

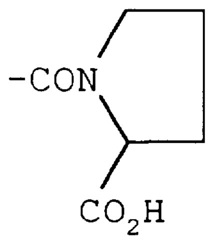

$R_4$ is preferably $CH_2$-$CH_2$-Y or CH=CH-Y (in either in the E or Z alkene stereochemical configuration) wherein Y is one of the preferred groups defined above.

Preferably $R_2$ is hydrogen or one of the groups specified above for $R_4$ .

R is preferably methyl, benzyl, p-methoxybenzyl, tert-butyl, diphenylmethyl, or hydrogen.

The present invention includes the salts of those compounds of formula (I) that have salt-forming groups, especially the salts of the compounds having a carboxylic group. The salt are physiologically tolerable salts, especially salts with alkali metals such as sodium, potassium and lithium; alkaline earth metals such as calcium and magnesium; ammonium; and salts with organic amine or amino acid (e.g. procaine, arginine).

Specific examples of the preferred compounds of the present invention are listed in Table 1 below:

TABLE 1

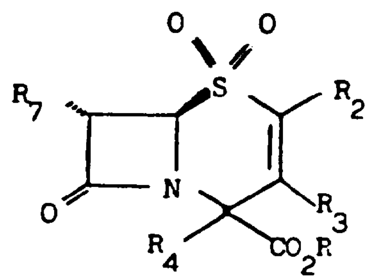

| * | $R_7$ | $R_2$ | $R_3$ | $R_4$ | R |
|---|---|---|---|---|---|
| 1 | Cl | $CH_2CH_2COCH_3$ | $CH_2OCOCH_3$ | $CH_2CH_2COCH_3$ | $CH_2-C_6H_4$-p-OMe |
| 2 | Cl | H | $CH_2OCOCH_3$ | $CH_2CH_2COCH_3$ | $CH_2-C_6H_4$-p-OMe |
| 3 | Cl | H | $CH_3$ | (E) $CH{=}CHCO_2Bu^t$ | $CH_3$ |
| 4 | Cl | H | $CH_3$ | (Z) $CH{=}CHCO_2Bu^t$ | $CH_3$ |
| 5 | Cl | (E) $CH{=}CHCO_2Bu^t$ | $CH_3$ | (Z,E) $CH{=}CHCO_2Bu^t$ | $CH_3$ |
| 6 | OMe | (E) $CH{=}CHCO_2Bu^t$ | $CH_3$ | (E) $CH{=}CHCO_2Bu^t$ | $CH_3$ |
| 7 | Cl | (E) CH=CHCON 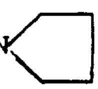 | $CH_3$ | (E) CH=CHCON 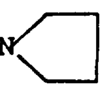 | $CH_3$ |
| 8 | Cl | H | $CH_3$ | (E) CH=CHCON 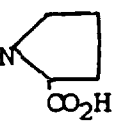 | $CH_2-C_6H_5$ |
| 9 | Cl | H | $CH_3$ | $CH_2CH_2COCH_3$ | $CH(C_6H_5)_2$ |

The present invention also provides a process for preparing compounds of formula (I), which process comprises

(1) reacting a compound of formula (II):

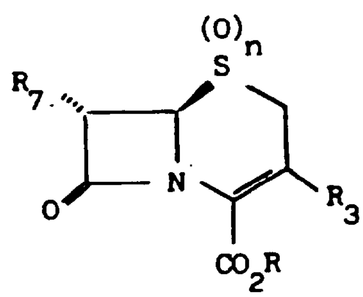

(II)

wherein R, $R_3$, and $R_7$ are as defined above, and n is 0, 1 or 2, with a compound of formula (III) or (IV)

$$CHR'{=}CR'Y \quad (III) \qquad CR'{\equiv}CY \quad (IV)$$

wherein R', R" and Y are as defined above, and, if n is 0 or 1, oxidizing the sulphur atom at the 1-position of the cephem nucleus to the sulphone level; and

(2) if desired, converting the resulting compound of formula (I) into a pharmaceutically or veterinary acceptable salt thereof.

The reaction of compounds of formula (II), as defined above, with the compounds of formula (III) or (IV), as defined above, is carried out in aprotic organic solvents such as dichloromethane, chloroform, acetonitrile, dimethylformamide, acetone, tetrahydrofuran, dioxane, ethyl acetate, dimethoxyethane, dichloroethane, dimethylsufoxide and benzene. Alternatively, the reaction can be carried out using as solvent an excess of the reagent (III) or (IV). Preferably the reaction temperature is between -20°C and +60°C, especially between 0°C and +25°C.

The reaction is usually run in the presence of an organic or inorganic base, in particular triethylamine (TEA), N-ethyldiisopropylamine, N,N-dimethylaniline, pyrridine, lutidine, collidine, 4-(N,N-dimethylamino)-pyridine (DMAP), 1,4-diazabicyclo[2.2.2]octane (DBO), 1,5-diazabicyclo[4.3.0]nonene (DBN), 1,8-diazabicyclo[5.4.0]undecene (DBU), 1,1,3,3-tetramethylguanidine, N-methylpyrrolidine, N-methylpiperidine or N-methylmorpholine, or potassium tert-butoxide, potassium carbonate, cesium carbonate, sodium carbonate, silver oxide, silver carbonate or silver fluoride. Instead of using such organic or inorganic bases, the reaction may be run in the presence of neutral or basic alumina.

In the process described above any functional group, if needed or desired, can be masked by conventional methods and unmasked at the end by conventional methods. Also, a group R, $R_3$, $R_4$ can be converted by conventional methods into a different group R, $R_3$, $R_4$, respectively, included within those previously defined, at the end or at any stage of the process above.

$\Delta^3$-Cephems of formula (II), substituted alkenes of formula (III) and substituted alkynes of formula (IV) are known compounds or can be prepared from known compounds by known methodologies.

The compounds of the present invention are characterised by high inhibitory activity on human leukocyte elastase (HLE), as is shown in Comparative Example 1 which follows.

It will be seen that the novel class of $\Delta^2$-cephem sulphones described by formula (I) includes very good inhibitors of HLE, i.e. compounds which are characterized by high "potency" (low apparent dissociation constant of the HLE-inhibitor complex at steady state, $K_i$*) and high "efficiency" (high rate of formation of HLE-inhibitor complex, $k_5/K_i$).

Compounds of formula (I) are also characterised by quite negligible levels of toxicity. Accordingly, they can be used in the treatment of inflammatory and degenerative diseases caused by proteolytic enzymes in mammals, including humans. The compounds can be used to make medicaments useful to prevent or arrest the progression of diseases caused by proteolytic degradation of lungs and connective tissues, reduce inflammation and fever, and relieve pain. Such diseases include emphysema, acute respiratory distress syndrome, bronchial inflammation, rheumatoid arthritis, osteoarthritis, infectious arthritis, rheumatic fever, spondylitis, gout, lupus, psoriasis, and the like.

The present invention also provides pharmaceutical and veterinary compositions comprising a pharmaceutically acceptable carrier and/or diluent and, as an active principle, a compound of formula (I) or a pharmaceutically acceptable salt thereof. Such pharmaceutical or veterinary compositions may be prepared in a conventional way by employing conventional non-toxic pharmaceutical carriers or diluents in a variety of dosage forms and ways of administration. In particular, the compounds of formula (I) can be administered:

a) orally, for example as tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavoring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, maize starch or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby providing a sustained action over a longer period: For example, a time delay material such as glyceryl monostearate or distearate may be employed.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active material in admixture with excipients suitable for the manifacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing

or wetting agents may be naturally-occurring phosphatides, for example lecithin, or condensation products of an alkylene oxide with fatty acids, for example polyoxyethylene stearate, or condensation products of ethylene oxide with long-chain aliphatic alcohols, for example heptadecaethyleneoxycetanol, or condensation products of ethylene oxide with partial esters derived from fatty acids and hexitol anhydrides, for example polyoxyethylene sorbitan monooleate. Said aqueous suspensions may also contain one or more preservatives, for example ethyl or propyl p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspension may contain a thickening agent, for example beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules suitable for preparing an aqueous suspension by the addition of water provide the active ingredient in admixture with a dispersing or wetting agent, a suspending agent, and one or more preservatives. Suitable dispersing or- wetting and suspending agents are exemplified by those already mentioned above. Additional excipients, for example sweetening, flavouring and coloring agents may also be present.

Oil-in-water emulsions may be formulated, wherein the oily phase may be a vegetable oil, for example olive oil or arachis oil, or a mineral oil, for example liquid paraffin, or mixtures of these. Suitable emulsifying agents may be naturally-occurring gums, for example gum acacia or gum tragacanth, naturally-occurring phosphatides, and esters or partial esters derived from fatty acids and hexitol anhydrides, for example sorbitan monooleate, or condensation products of said partial esters with ethylene oxide, for example polyoxyethylene sorbitan monooleate. The emulsion may also contain sweetening and flavoring agents.

Syrups and elixirs may be formulated with sweetening agents, for example glycerol, sorbitol or sucrose. Such formulations may also contain a demulcent, a preservative, and flavoring and coloring agents;

b)parenterally, either subcutaneously, or intravenously, or intramuscularly, or intrasternally, or by infusion techniques, in the form of sterile injectable aqueous or oleagenous suspensions. The suspension may be formulated according to the known art using dispersing or wetting and suspending agents such as those mentioned above. The sterile injectable preparation may also be a sterile injectable solution or suspension in a parenterally acceptable diluent or solvent, for example as a solution in 1,3-butanediol. Among the acceptable vehicles and solvents that may be employed are water, Ringer's solution and isotonic sodium chloride solution. In addition sterile, fixed oils are conventionally employed as a solvent or suspending medium. For this purpose any bland fixed oil may be employed including synthetic mono- or diglycerides. In addition fatty acids such as oleic acid find use in the preparation of injectables;

c)by inhalation, in the form of aerosols or solutions for nebulizers;

d)rectally, in the form of suppositories prepared by mixing the drug with a suitable non-irritating excipient which is solid at ordinary temperatures but liquid at the rectal temperature and will melt in the rectum to release the drug. Such materials are cocoa butter and polyethylene glycols;

e) topically, in the form of creams, ointments, jellies, solutions or suspensions.

The present invention further provides a method of controlling inflammatory and degenerative diseases by administering a therapeutically effective amount of one or more active compounds of formula (I) to humans or mammalians in need of such treatment. Daily doses are typically in the range of about 0.5 to about 100 mg per kg of body weight, according to the activity of the specific compound, the age, weight and conditions of the subject to be treated, the type and severity of the disease, and the frequency and route of administration; preferably daily dosage levels for humans are in the range of 50 mg to 2 g. The amount of active ingredient that may be combined with the carrier materials to produce a single dosage form will vary depending upon the host treated and the particular mode of administration. For example, a formulation intended for the oral administration to humans may contain from 5 mg to 2 g of active ingredient compounded with a convenient amount of carrier material which may vary from about 5 to about 96 percent of the total composition. Dosage unit forms will generally contain from 25 mg to about 500 mg of active ingredient.

A further aspect of the invention is the use of a compound of formula (I) as hereinbefore defined, or a pharmaceutically or veterinary acceptable salt thereof, in the manufacture of a medicament for the treatment of inflammatory or degenerative diseases caused by proteolytic enzymes.

The invention will be further illustrated by the Examples which follow:

EXAMPLE 1

Benzhydryl-7α-chloro-3-methyl-4-(3-oxo-butyl)-2-cephem-4-carboxylate-1,1-dioxide (Compound 9)

Benzhydryl 7α-chloro-3-methyl-3-cephem-4-carboxylate-1,1-dioxide (665 mg) was dissolved in methyl vinyl ketone (2 ml). After cooling to 0°C, triethylamine (0.045 ml) was added and the solution was stirred at that temperature for 1 h. Following concentration under reduced pressure, the residue was purified by silica gel chromatography eluting with cyclohexane:ethyl acetate mixtures. The title product (470 mg) was obtained as a white powder.

IR (KBr) 1800, 1750, 1712 cm$^{-1}$
NMR ($CDCl_3$) δ 1.72 (3H, d, J≦ 1.1 Hz), 2.14(3H, s), 2.2-3.0(4H, m), 4.76(1H, d, J= 1.1 Hz), 5.26 (1H, d J= 1.1 Hz), 6.35(1H, d, J≦ 1.1 Hz), 6.93(1H, s), 7.2-7.5(10H, m).

EXAMPLE 2

p-Methoxybenzyl-3-acetoxymethyl-7α-chloro-4-(3-oxo-1-butyl)-2-cephem-4-carboxylate-1,1-dioxide (Compound 2), and p-Methoxybenzyl-3-acetoxymethyl-7α-chloro-2,4-bis(3-oxo-1-butyl)-2-cephem-4-carboxylatel-1,1-dioxide (Compound 1).

p-Methoxybenzyl 3-acetoxymethyl-7α-chloro-3-cephem-4-carboxylate-1,1-dioxide (2.89 g) was dissolved in dichloromethane (6 ml) and methyl vinyl ketone (6 ml). Triethylamine (0.29 ml) was added and the solution was stirred at room temperature for 1 h. Following concentration under reduced pressure, the residue was purified by silica gel chromatography eluting with cyclohexane:ethyl acetate mixtures. The title products Compound 2 (1.7 g) and Compound 1 (900 mg) were obtained as white foams.

Compound 2:

IR ($CHCl_3$) 1795, 1740, 1714 cm$^{-1}$
NMR ($CDCl_3$) δ 2.10(3H, s), 2.14(3H, s), 2.2-3.0(4H, m), 3.81(3H, m), 4.52(1H,dd, J= 1.7 and 16.0 Hz), 4.69(1H, d, J= 1.6 Hz), 4.70 (1H, dd, J= 1.7 and 16 Hz), 5.14 (1H, d, J= 11.6 Hz), 5.23 (1H, d, J= 1.6 Hz), 5.27(1H, d, J= 11.6 Hz), 6.56(1H, t, J= 1.7 Hz), 6.89(2H, d. J= 8.7 Hz), 7.27(2H, d, J= 8.7 Hz).

Compound 1:

IR ($CHCl_3$) 1793, 1740-1710 cm$^{-1}$
NMR ($CDCl_3$) δ 1.97 (3H, s), 2.13(3H, s), 2.15(3H, s), 2.2-3.0 (4H, m), 3.81(3H, s), 4.65(1H, d, J= 1.4 Hz), 4.73(2H, ABq, J= 13.6 Hz), 5.05 (1H, d, J= 11.7 Hz), 5.21(1H, d, J= 1.4 Hz), 5.25 (1H, d, J= 11.7 Hz), 6.89(2H, d, J= 8.7 Hz), 7.27(2H, d, J= 8.7 Hz).

EXAMPLE 3

Methyl-7α-Chloro-2,4-bis[2-tert-butoxycarbonylethenyl]-3-methyl-2-cephem-4-carboxylate-1,1-dioxide (Compound 5); Methyl 7α-chloro-4-[(*Z*)-2-tert-butoxycarbonylethenyl]-3-methyl-2-cephem-4-carboxylate-1,1-dioxide (Compound 4); and Methyl 7α-chloro-4-[(*E*)-2-tert-butoxycarbonylethenyl-3-methyl-2-cephem-4-carboxylate-1,1-dioxide (Compound 3).

A solution of methyl 7α-chloro-3-methyl-3-cephem-4-carboxylate-1,1-dioxide (1.1 g) in dichloromethane (1 ml) and tert-butyl propiolate (2 ml) was treated with triethylamine (0.20 ml). After stirring for 4 h, the mixture was concentrated under reduced pressure and the residue was fractionated by silica gel chromatography (cyclohexane:ethyl acetate mixtures). Compound 5 (ca. 2:1 mixture of *Z.E* and *E.E* alkene isomers by NMR) was eluted first and obtained as a white powder (380 mg):

IR (KBr) 1810, 1718 cm$^{-1}$.
NMR ($CDCl_3$) Major isomer: δ 1.44, 1.49(each 9H, s), 1.86(3H, s), 3.85(3H, s), 5.25, 5.41(each 1H, d, J= 1.5 Hz), 6.00( 1H, d, J= 12.7 Hz), 6.19(1H, d, J= 16.0 Hz), 6.33(1H, d, J= 12.7 Hz), 6.76 (1H, d, J= 16.0 Hz). Minor isomer: δ 1.53, 1.56(each 9H, s), 1.84 (3H, s), 3.92(3H, s), 4.93, 5.30(each 1H, d, J= 1.5 Hz), 5.87, 6.23(each 1H, d, J=

16.0 Hz), 6.81, 7.08(each 1H, d, J= 16.0 Hz).

Compound 4 was the second-eluted fraction (white powder, 78 mg):

IR (KBr) 1802, 1740, 1716 $cm^{-1}$;
NMR ($CDCl_3$) δ 1.46(9H, s), 2.02(3H, d, J= 1.5 Hz), 3.82(3H, s), 5.05(1H, d, J= 1.4 Hz), 5.26(1H, d, J= 1.4 Hz), 5.96 (1H, d, J= 11.7 Hz), 6.28(1H, d, J= 11.7 Hz), 6.31(1H, d, J= 1.5 Hz).

Compound 3 was the third-eluted fraction (white powder, 160 mg):

IR (KBr) 1804, 1745, 1710 $cm^{-1}$;
NMR ($CDCl_3$) δ 1.48(9H, s), 1.90(3H, d, J= 1.4 Hz), 3.92(3H, s), 4.84(1H, d, J= 1.6 Hz), 5.30(1H, d, J= 1.6 Hz), 6.09 (1H, d, J= 16.0 Hz), 6.39(1H, d, J= 1.4 Hz), 7.27(1H, d, J= 16.0 Hz).

Comparative Example 1

The inhibitory activity on human leukocyte elastase (HLE) of $\Delta^2$-cephem sulphones of the invention, designated as Compound 4 and Compound 5 in Table 1 above, and of the $\Delta^3$-cephem sulphone Merck S.&D. L-659,286, which, to our best knowledge, is the most advanced β-lactam product under study as an HLE-inhibitor (Ann. Rev. Resp. Dis. 1988, 137:204; Journal of Cellular Biochemistry 1989, 39:47), was compared as described below.

The reference compound 7α-methoxy-3-(2-methyl-5-oxo-6-hydroxy-2,5-dihydro-1,2,4-triazin-3-yl)thiomethyl-3-cephem-4-pyrrolidine-carboxamide-1,1-dioxide (Merck's code-name; L-659,286) was synthesized in our laboratories from 7-amino-3-desacetoxy-cephalosporanic acid. Its structural identity and purity (≥95%) was confirmed by spectral and analytical data.

The kinetic parameters of HLE (Calbiochem) inhibition were determined at 37°C in phosphate buffer (pH 7.4, I= 0.15) with 1% DMSO and 1% MeCN as solubilizing vehicles, monitoring fluorometrically ($\lambda_{ex}$= 383 nm; $\lambda_{em}$ = 455 nm) the release of 7-amino-4-methylcoumarin from the fluorogenic substrate N-methoxysuccinyl-alanyl-alanyl-prolyl-valyl-7-amido-4-methylcoumarin. Best fit to the progress curves was obtained by a two-step mechanism (mechanism B in J.F. Morrison, TIBS 1982, 7:102; see also J.F. Morrison and C.T. Walsh, Advances in Enzymology, vol. 61, A. Meister editor, John Wiley & Sons 1988):

$$E + I \underset{k_4}{\overset{k_3}{\rightleftharpoons}} EI \underset{k_6}{\overset{k_5}{\rightleftharpoons}} EI^*$$

Product release versus time was analysed using the following function:

$$[P] = v_s t + (v_i - v_s)(1 - e^{-kt})/k$$

wherein [P] = product concentration, $v_s$ = steady state rate, vi = initial rate of product release and

$$k = k_6 + (k_5/K_i)\frac{[I]}{1 + [S]/K_m + [I]/K_i};\ K_i = k_4/k_3$$

wherein [I] and [S] = inhibitor and substrate concentration, and $K_m$ = Michaelis constant for the enzymesubstrate pair (independently determined under the same experimental conditions). At steady state, inhibition is fully determined by the steady-state inhibition constant $K_i$* (steady state):

$$K_i^* = K_i \frac{k_6}{k_5 + k_6}$$

The results were as follows:

TABLE 2

| | |
|---|---|
| Compound n°4 | $k_5/K_i$ = 7,200 $M^{-1}s^{-1}$<br>$K_i$* (steady state) = 64 nM |
| Compound n°5 | $k_5/K_i$ = 70,000 $M^{-1}s^{-1}$<br>$K_i$* (steady state) = 5.2 nM |
| Reference compound | $k_5/K_i$ = 8,000 $M^{-1}s^{-1}$<br>$K_i$* (steady state) = 140 nM |

**Claims**

1. A Δ2-cephem sulphone of formula (I) or a pharmaceutically or veterinary acceptable salt thereof;

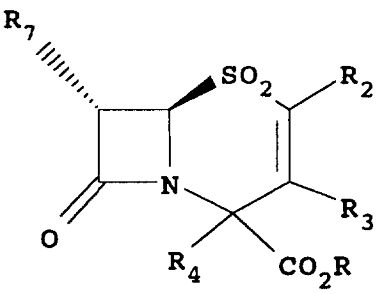

wherein $R_7$ is a halogen or a $C_1$-$C_4$ alkoxy group; $R_4$ is a substituted alkyl group CHR'-CH"Y, or an alkenyl group CR'=CHY, wherein R' and R" each represent, independently, hydrogen or an unsubstituted or substituted with free carboxy group, $C_1$-$C_7$ straight or branched alkyl, phenyl or benzyl group or R' or R", taken together with the carbon atoms to which they are attached, form a five membered or six membered ring of the formulae

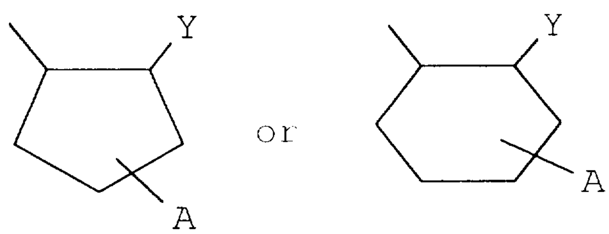

wherein Y is acyl C(O)R' wherein R' is as defined above; alkoxycarbonyl and benzyloxycarbonyl C(O)OR' wherein R' is as defined above ;
alkylsulphinyl, phenylsulphinyl and benzylsulphinyl S(O)R' wherein R'is as defined above;
alkylsulphonyl, phenylsulphonyl, and benzylsulphonyl $S(O)_2R'$ wherein R' is as defined above;
carbamoyl C(O)NR'R" and aminosulphonyl S(O)NR'R" wherein R' and R" are as defined above or wherein R' and R", taken together with the nitrogen atom to which they are attached, form an unsubstituted or substituted with free carboxy group, pyrrolidine or piperidine ring; a nitro group or a cyano group and A is hydrogen or a carboxy group; R is hydrogen, a $C_1$-$C_7$ straight or branched alkyl, p-methoxybenzyl, benzyl or diphenylmethyl group; $R_3$ is methyl or a $C_1$-$C_4$ alkoxymethyl group; and $R_2$ is hydrogen or a group $R_4$ as defined above.

2. A compound according to claim 1 in which $R_7$ is chlorine or a methoxy group; Y is an acetyl, pivaloyl, benzoyl, phenylacetyl,methoxycarbonyl, tert-butoxycarbonyl, benzyloxycarbonyl, carboxy, cyano, nitro, mesyl, tosyl, phenylsulfonyl, tert-butylsulfonyl or benzylsulfonyl group, or a carbamoyl derivative of formula

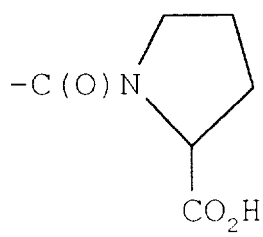

$R_4$ represents CH-CH-Y or CH=CH-Y, either in the E or Z alkene stereochemical configuration, wherein Y is as defined in claim 1;
$R_2$ is hydrogen or is as defined above for $R_4$;
R is hydrogen or a methyl, benzyl, p-methoxybenzyl, tert-butyl or diphenylmethyl group; and
R' and R" each represent independently a $C_1$-$C_7$ straight or branched alkyl group selected from methyl, ethyl, i-propyl and tert-butyl.

3. A process for the preparation of a compound of formula (I), as defined in claim 1, or a pharmaceutically or veterinary acceptable salt thereof, which process comprises reacting a compound of formula (II)

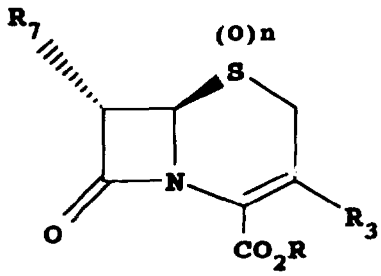

(II)

wherein n is 0,1 or 2 and R, $R_3$ and $R_7$ are as defined in claim 1, with a compound of the formula (III) or (IV)

$$CHR'=CR''Y \quad (III)$$

$$CR'\equiv CY \quad (IV)$$

wherein R', R" and Y are as defined in claim 1, and, if needed, oxidizing, wherein n is 0 or 1, the sulphur atom, at the 1-position of the cephem nucleus, to the sulphone level; and/or converting, if desired, the resulting compound of formula (I) into a pharmaceutically or veterinarily acceptable salt thereof.

4. A process according to claim 3 wherein the reaction of the compound of formula (II) with the compound of formula (III) or (IV), is carried out in the presence of an organic or inorganic base, or a neutral or basic alumina, in an aprotic organic solvent or in, as solvent, an excess of the compound (III) or (IV), at a temperature of from -20°C to +60°C.

5. A pharmaceutical or veterinary composition comprising a pharmaceutically acceptable carrier and/or diluent and, as an active principle, a compound as claimed in claim 1 or 2 or a pharmaceutically or veterinarily acceptable salt thereof.

6. A compound according to claim 1 or 2 or a pharmaceutically or veterinarily acceptable salt thereof, for use in the treatment of inflammatory and degenerative diseases caused by proteolytic enzymes in mammals including humans.

7. A compound according to claim 6 for use in the treatment of emphysema, adult respiratory distress syndrome, rheumatic fever, spondylitis, gout, lupus or psoriasis.

8. Use of a compound as claimed in claim 1 or 2, or a pharmaceutically or veterinarily acceptable salt thereof, in the manufacture of a medicament for the treatment of inflammatory or degenerative diseases caused by proteolytic enzymes.

**Patentansprüche**

1. Ein Δ2-Cephemsulfon der Formel (I) oder ein pharmazeutisch oder tierärztlich annehmbares Salz desselben

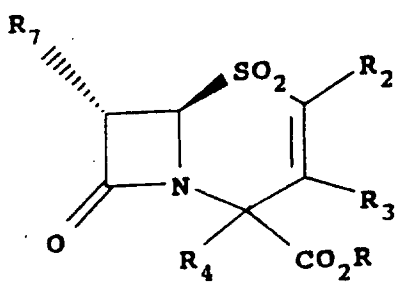

worin $R_7$ eine Halogen- oder eine $C_1$-$C_4$-Alkoxygruppe darstellt; $R_4$ eine substituierte Alkylgruppe der Formel CHR'-CH"Y oder eine Alkenylgruppe der Formel CR'=CHY bedeutet, worin R' und R", jeweils unabhängig voneinander, Wasserstoff oder eine Gruppe, die nicht substituiert oder mit einer freien Karboxygruppe substituiert ist, eine geradkettige oder verzweigte $C_1$-$C_7$-Alkyl-, Phenyl- oder Benzylgruppe darstellen oder R' und R",

zusammen mit den Kohlenstoffatomen, an welchen sie anhaften, einen fünfgliedrigen oder sechsgliedrigen Ring mit der Formel

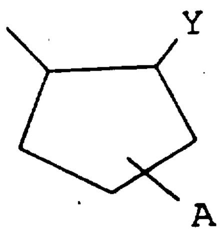

oder

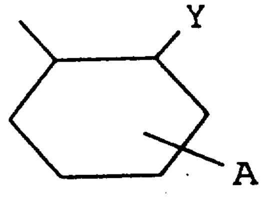

bilden, worin Y bedeutet: Acyl der Formel C(O)R', worin R' die obenangegebene Bedeutung hat; Alkoxycarbonyl und Benzyloxycarbonyl der Formel C(O)OR', worin R' die obenangegebene Bedeutung hat; Alkylsulfinyl, Phenylsulfinyl und Benzylsulfinyl der Formel S(O)R', worin R' die obenangegebene Bedeutung hat; Alkylsulfonyl, Phenylsulfonyl und Benzylsulfonyl der Formel $S(O)_2R'$, worin R' die obenangegebene Bedeutung hat; Carbamoyl der Formel C(O)NR'R" und Aminosulfonyl der Formel $S(O)_2NR'R"$, worin R' und R" die obenangegebene Bedeutung haben oder worin R' und R", zusammen mit dem Stickstoffatom, an welchem sie anhaften, eine Gruppe, die nicht substituiert oder mit einer freien Carboxygruppe substituiert ist, einen Pyrrolidin- oder Piperidinring bilden; eine Nitrogruppe oder eine Zyangruppe und A Wasserstoff oder eine Carboxygruppe darstellen; R Wasserstoff, eine geradkettige oder verzweigte $C_1$-$C_7$-Alkyl-, p-Methoxybenzyl-, Benzyl- oder Diphenylmethylgruppe darstellen; R Methyl oder eine $C_1$-$C_4$-Alkoxymethylgruppe bedeutet; und $R_2$ Wasserstoff oder eine so wie oben definierte $R_4$ Gruppe darstellt.

**2.** Eine Verbindung gemäss Patentanspruch 1, dadurch gekennzeichnet, dass $R_7$ Chlor oder eine Methoxygruppe bedeutet; Y eine Acetyl-, Pivaloyl-, Benzoyl-, Phenylacetyl-, Methoxycarbonyl-, tert-Butoxycarbonyl-, Benzyloxycarbonyl-, Carboxy-, Zyan-, Nitro-, Mesyl-, Tosyl-, Phenylsulfonyl-, tert-Butylsulfonyl- oder Benzylsulfonylgruppe, oder ein Carbamoylderivat der Formel

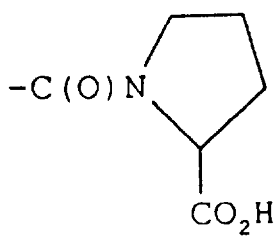

darstellt;

$R_4$ $CH_2$-$CH_2$-Y oder CH=CH-Y darstellt, entweder in der E- oder Z-alkenstereochemischen Konfiguration, worin Y die im Patentanspruch 1 angegebene Bedeutung hat;
$R_2$ Wasserstoff darstellt oder die für $R_4$ obenangegebene Bedeutung hat;
R Wasserstoff oder eine Methyl-, Benzyl-, p-Methoxybenzyl-, tert-Butyl- oder Diphenylmethylgruppe bedeutet; und R' und R", jeweils unabhängig voneinander, eine unter Methyl, Äthyl, i-Propyl und tert-Butyl ausgewählte geradkettige oder verzweigte $C_1$-$C_7$-Alkylgruppe darstellen.

**3.** Ein Verfahren zur Herstellung einer Verbindung der Formel (I), wie sie im Patentanspurch 1 definiert ist, oder ein pharmazeutisch oder tierärztlich annehmbares Salz derselben, dadurch gekennzeichnet, dass es die Reaktion einer Verbindung der Formel (II)

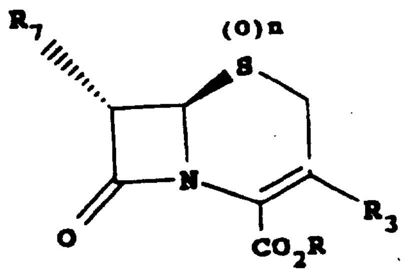

(II)

worin n für 0, 1 oder 2 steht und R, $R_3$ und $R_7$ die im Patentanspruch 1 definierten Bedeutungen haben, mit einer Verbindung der Formel (III) oder (IV)

$$CHR'=CR''Y \quad (III)$$

$$CR'\equiv CY \quad (IV)$$

worin R', R" und Y die im Patentanspruch 1 angegebenen Bedeutungen haben, sowie, wenn erforderlich, die Oxydation, worin n für 0 oder 1 steht, des Schwefelatoms an der 1-Stellung des Cephemkerns auf die Sulfonebene; und/oder die Konversion, sofern dies gewünscht wird, der sich ergebenden Verbindung der Formel (I) in ein pharmazeutisch oder tierärztlich annehmbares Salz derselben, umfasst.

4. Verfahren gemäss Patentanspruch 3, dadurch gekennzeichnet, dass die Reaktion der Verbindung der Formel (II) mit der Verbindung der Formel (III) oder (IV) in Anwesenheit einer organischen oder inorganischen Base oder einem neutralen oder basischen Aluminiumoxyd, in einem aprotischen organischen Lösungsmittel oder in einem, als Lösungsmittel angewendeten Überschuss der Verbindung (II) oder (IV), bei einer Temperatur von -20°C bis +60°C erfolgt.

5. Eine pharmazeutishce oder tierärztliche Zusammensetzung, dadurch gekennzeichnet, dass sie einen pharmazeutisch annehmbaren Träger und/oder Verdünner und, als Wirkstoff, eine wie in den Patentansprüchen 1 oder 2 beanspruchte Verbindung oder ein pharmazeutisch oder tierärztlich annehmbares Salz derselben enthält.

6. Eine Verbindung gemäss Patentanspruch 1 oder 2, oder ein pharmazeutisch oder tierärztlich annehmbares Salz derselben, dadurch gekennzeichnet, dass man sie zur Behandlung von entzündlichen oder degenerativen, durch proteolytische Enzyme verursachten Krankheiten bei Säugern, einschliesslich des Menschen anwendet.

7. Eine Verbindung gemäss Patentanspruch 6, dadurch gekennzeichnet, dass man sie zur Behandlung von Emphysemen, Atemnotsyndromen der Erwachsenen, rheumatischem Fieber, Spondylitis, Gicht, Lupus oder Psoriasis anwendet.

8. Anwendung einer so wie in den Patentansprüchen 1 oder 2 beanspruchten Verbindung, oder eines pharmazeutisch oder tierärztlich annembaren Salzes derselben, zur Herstellung eines Arzneimittels zur Behandlung von entzündlichen oder degenerativen, durch proteolytische Enzyme bedingten Krankheiten.

## Revendications

1. Un Δ2-céphem sulfone représenté par la formule (I) ou l'un de ses sels acceptables du point de vue pharmaceutique ou vétérinaire

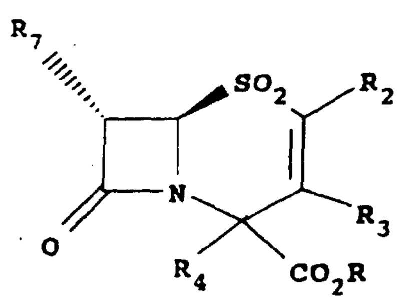

dans laquelle $R_7$ représente un groupe halogène ou un groupe $C_1$-$C_4$-alkoxy; $R_4$ représente un groupe alkyle substitué de formule CHR'-CH"Y, ou un groupe alkényle de formule CR'=CHY où R' et R" représentent, indépendamment l'un de l'autre, hydrogène ou un groupe non substitué ou substitué par un groupe carboxy libre, un groupe linéaire ou ramifié $C_1$-$C_7$-alkyle, phényle ou benzyle ou R' et R", pris ensemble avec l'atome de carbone, auquel ils sont attachés, forment un anneau à cinq ou à six éléments ayant la formule

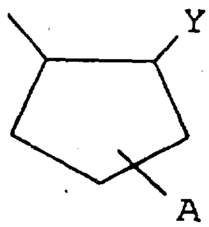

ou

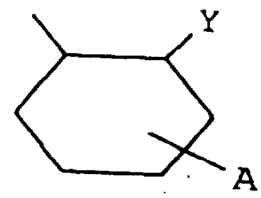

dans laquelle Y représente acyle de formule C(O)R', où R' est tel que défini ci-dessus;
alkoxycarbonyle et benzyloxycarbonyle de formule C(O)OR', où R' est tel que défini ci-dessus;
alkylsulfinyle, phénylsulfinyle et benzylsulfinyl de formule S(O)R', où R' est tel que défini ci-dessus;
alkylsulfonyle, phénylsulfonyle et benzylsulfonyle de formule $S(O)_2R'$, où R' est tel que défini ci-dessus;
carbamoyle de formule C(O)NR'R" et aminosulfonyle de formule $S(O)_2NR'R"$, où R' et R" sont tels que définis ci-dessus ou dans lequel R' et R", pris ensemble avec l'atome de nitrogène, auquel ils sont attachés, forment un groupe non substitué ou substitué par un groupe carboxy libre, un anneau pyrrolidinique ou un anneau pipéridinique; un groupe nitro ou cyano et A représente hydrogène ou un groupe carboxy; R représente hydrogène, un groupe linéaire ou ramifié $C_1$-$C_7$-alkyle, p-méthoxybenzyle, benzyle ou diphénylméthyle; $R_3$ représente méthyle ou un groupe $C_1$-$C_4$-alkoxyméthyle; et $R_2$ représente hydrogène ou un groupe $R_4$ tel que défini ci-dessus.

**2.** Un composé selon la revendication 1, caractérisé par le fait que $R_7$ représente chlore ou un groupe méthoxy; Y représente un groupe acétyle, pivaloyle, benzoyle, phénylacétyle, méthoxycarbonyle, tert-butoxycarbonyle, benzyloxycarbonyle, carboxy, cyano, nitro, mésyle, tosyle, phénylsulfonyle, tert-butylsulfonyle ou benzylsulfonyle, ou un dérivé de carbamoyle ayant la formule

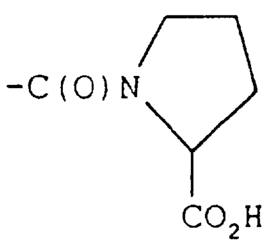

$R_4$ représente $CH_2$-$CH_2$-Y ou CH=CH-Y, dans la configuration stéréochimique alkène E ou Z, où Y est tel que défini à la revendication 1;
$R_2$ représente hydrogène ou bien a la signification indiqueée ci-dessus pour $R_4$;
R représente hydrogène ou un groupe méthyle, benzyle, p-méthoxybenzyle, tert-butyle ou diphénylméthyle; et R' et R" représentent, indépendamment l'un de l'autre, un groupe linéaire ou ramifié $C_1$-$C_7$-alkyle, choisi parmi méthyle, éthyle, i-propyle et tert-butyle.

**3.** Un procédé de fabrication d'un composé représenté par la formule (I), telle que définie à la revendication 1, ou de l'un de ses sels acceptables du point de vue pharmaceutique ou vétérinaire, caractérisé par le fait qu'il comprend la réaction d'un composé de la formule (II)

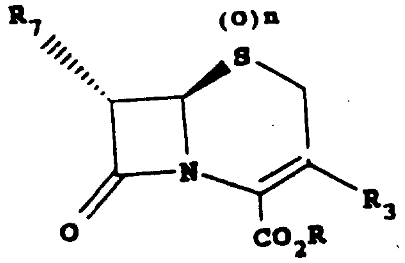

(II)

dans laquelle n est 0, 1 ou 2 et R, $R_3$ et $R_7$ sont tels que définis à la reventication 1, avec un composé représenté par la formula (III) ou (IV)

CHR'=CR"Y (III)

CR'≡CY (IV)

dans laquelle R', R" et Y sont tels que définis à la revendication 1, et, si nécessaire, l'oxydation, où n est 0 ou 1, de l'atome sulfurique à la position 1 du noyau céphem au niveau sulfonique; et/ou, pour autant qu'on le souhaite, la conversion du composé résultant de la formule (I) en l'un de ses sels acceptables du point de vue pharmaceutique ou vétérinaire.

**4.** Un procédé selon la revendication 3, caractérisé par le fait qu'on effectue la réaction du composé représenté par la formule (II) avec un composé représenté par la formule (III) ou (IV) en présence d'une base organique ou inorganique, ou d'une alumine neutre ou basique, dans un solvant organique aprotique ou dans un excédent du composé (III) ou (IV) utilisé comme solvant, à une température de -20°C à +60°C.

**5.** Une composition pharmaceutique ou vétérinaire, caractérisée par le fait qu'elle contient un support et/ou diluant acceptable du point de vue pharmaceutique et, comme principe actif, un composé tel que revendiqué à la revendication 1 ou 2, ou l'un de ses sels acceptables du point de vue pharmaceutique ou vétérinaire.

**6.** Un composé selon la revendication 1 ou 2, ou l'un de ses sels acceptables du point de vue pharmaceutique ou vétérinaire, caractérisé par le fait que l'on l'emploie pour le traitement des maladies inflammatoires et dégénérative provoquées par des enzymes protéolytiques chez les mammifères, y inclus l'homme.

**7.** Un composé selon la revendication 6, caractérisé par le fait que l'on l'emploie pour le traitement des emphysèmes, de la syndrome de dyspnée chez l'adulte, de la fièvre rhumatique, de la spondylite, de la goutte, du lupus ou de la psoriasis.

**8.** Emploi d'un composé tel que revendiqué à la revendication 1 ou 2, ou de l'un de ses sels acceptables du point de vue pharmaceutique ou vétérinaire, pour la préparation d'un médicament pour le traitement des maladies inflammatoires ou dégénératives provoquées par des enzymes protéolytiques.